(19) Europäisches Patentamt
European Patent Office
Office européen des brevets

(11) **EP 1 859 811 B1**

(12) **EUROPEAN PATENT SPECIFICATION**

(45) Date of publication and mention
of the grant of the patent:
**24.08.2011 Bulletin 2011/34**

(51) Int Cl.:
*A61K 47/48* *(2006.01)*    *A61P 35/00* *(2006.01)*
*A61P 29/00* *(2006.01)*

(21) Application number: **06010902.2**

(22) Date of filing: **27.05.2006**

(54) **Use of conjugates of amatoxins or phallotoxins with macromolecules for tumor and inflammation therapy**

Anwendung von Amatoxin-Konjugaten und Phallotoxin-Konjugaten mit Makromolekülen zur Krebstherapie und Therapie von Entzündungen

Application de conjuguées d' amatoxines et de phallotoxines avec des macromolécules pour la thérapie du cancer et de l'Inflammation

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**28.11.2007 Bulletin 2007/48**

(73) Proprietors:
• **Faulstich, Heinz, Dr.**
**69123 Heidelberg (DE)**
• **Deutsches Krebsforschungszentrum**
**69009 Heidelberg (DE)**

(72) Inventors:
• **Faulstich, Heinz**
**69123 Heidelberg (DE)**
• **Faulstich, Dorian**
**68542 Heddesheim (DE)**
• **Anderl, Jan Dr.**
**64397 Modautal (DE)**
• **Moldenhauer, Gerhard Dr.**
**D-69120 Heidelberg (DE)**

(74) Representative: **Zwicker, Jörk et al**
**Dr. Volker Vossius**
**Patent- und Rechtsanwaltskanzlei**
**Geibelstrasse 6**
**81679 München (DE)**

(56) References cited:
EP-A- 0 353 960     WO-A-2004/043461
US-A- 5 378 688     US-A1- 2004 192 900
US-B1- 6 706 713

• **BERMBACH U AND FAULSTICH H.: "Epidermal growth factor labeled beta-amanitin-poly-L-ornithin: preparation and evidence for specific cytotoxicity" BIOCHEMISTRY, vol. 29, 1990, pages 6839-6845, XP002404130**
• **FAULSTCIH, H; TRISCHMANN, H; WIELAND, TH: "Pept., Proc. Eur. Pept. Symp., 13th" 1975, WILEY , NEW YORK 00 , XP008053982 Pag. 333 par. 1-3; pag. 334 Fig. 2; pag. 337, line 9 -line 11 \* page 337, line 9 - line 11 \***
• **FAULSTICH H ET AL: "Protein conjugates of fungal toxins" 1985, METHODS IN ENZYMOLOGY 1985 UNITED STATES, VOL. 112, PAGE(S) 225-237 , XP008053933 Table 1 page 229; table 3 pag. 234**
• **BARBANTI BRODANO G ET AL: "Selective killing of macrophages by amanitin albumin conjugates" NATURE NEW BIOL. 1973, vol. 243, no. 130, 1973, pages 281-283, XP008053926**

Remarks:
The file contains technical information submitted after the application was filed and not included in this specification

**Description**

Amatoxins (α-amanitin, β-amanitin, amanin) are cyclic peptides composed of 8 amino acids. They can be isolated from *Amanita phalloides* mushrooms or prepared from the building blocks by synthesis. Amatoxins inhibit specifically the DNA-dependent RNA polymerase II of mammalian cells, and by this transcription and protein biosynthesis of the cells affected. Inhibition of transcription in a cell causes stop of growth and proliferation. Though not covalently bound, the complex between amanitin and RNA-polymerase II is very tight ($K_D$ = 3nM). Dissociation of amanitin from the enzyme is a very slow process what makes recovery of an affected cell unlikely. When in a cell the inhibition of transcription will last too long, the cell undergoes programmed cell death (apoptosis), as shown in cultures of Jurkat cells incubated with α-amanitin, or, with much higher efficiency, in Jurkat cells incubated with the membrane-permeable O-methyl-α-amanitin oleate (own results, unpublished).

Phallotoxins (phalloidin, phallacidin, aminophalloidin) are cyclic peptides composed of 7 aminoacids. They bind specifically to the cytoskeletal protein actin in its polymeric form (microfilaments). By this, they prevent depolymerization of the microfilaments and disturb the proper function of the actin cytoskeleton. The actin cytoskeleton is a dense meshwork of microfilaments undergoing, under the control of actin-associated proteins, rapid cycles of assembly and disassembly essential for dynamic processes such as cell division and migration. Likewise, invasion by metastatic cells is thought to be dependent on actin. In cancer cells, structural and functional perturbations of the actin cytoskeleton may correlate with higher proliferation rates and uncontrolled movement. Molecules that act on the cytoskeleton of tumor cells inhibit cell division and cause programmed cell death. Membrane-permeable phallotoxins like phalloidin oleate caused apoptosis in cultures of Jurkat cells, as shown in our laboratory (unpublished).

Permeation rates of native amatoxins and phallotoxins through cell membranes are low. By this reason most kinds of mammalian cells are protected from amatoxins and phallotoxins. However, parenchymal cells of liver possess transporting systems (organic anion transporting proteins, OATP), which facilitate the uptake of the toxins into hepatocytes. It is by the presence of these transporting systems in liver that in human Amanita mushroom poisoning mainly hepatocytes are affected, and that hepatocytes of e.g. mouse, rat and human were, so far, the models most frequently used for studying the biological activities of amatoxins and phallotoxins. Once declared as hepatotoxins, the conspicuous liver toxicity of the amatoxins and phallotoxins so far discouraged all plans to use the toxins as drugs in tumor therapy.

According to the present invention, amatoxins and phallotoxins can be converted to drugs for tumor therapy by covalently linking them to macromolecules. The resulting toxin conjugates are composed of 2 components

Carrier-Toxin

**Toxin** is selected from amatoxins.
Amatoxins are all cyclic peptides composed of 8 amino acids as isolated from the mushroom *Amanita phalloides* and described in ref. (Wieland, T. and Faulstich H., Crit. Rev. Biochem. 5, (1978), 185-260); further all chemical derivatives thereof; further all semisynthetic analogs thereof; further all synthetic analogs thereof built from building blocks according to the master structure of the natural compounds (cyclic, 8 aminoacids). Functionally, amatoxins are defined as peptides or depsipeptides that inhibit mammalian RNA polymerase II. Preferred amatoxins are those with a functional group (e.g. a carboxylic group, an amino group, a thiol or a thiol-capturing group) that can be reacted with linker or carrier molecules.

Other toxins known in the art are phallotoxins. Phallotoxins are all cyclic peptides composed of 7 aminoacids as isolated from the mushroom *Amanita phalloides* and described in ref. (Wieland, T. and Faulstich H., Crit. Rev. Biochem. 5, (1978), 185-260). Further all chemical derivatives thereof; further all semisynthetic analogs thereof; further all synthetic analogs thereof built from building blocks according to the master structure of the natural compounds (cyclic, 7 aminoacids). Functionally, phallotoxins are defined as peptides or depsipeptides that bind to polymeric actin and inhibit depolymerisation of microfilaments. Preferred phallotoxins are those with a functional group (e.g. a carboxylic group, an amino group, a thiol or a thiol-capturing group) that can be reacted with linker or carrier molecules.

The carrier comprises a tumor-specific monoclonal antibody which binds or is taken up by tumour cells.

Preferred carriers have a molar mass from 20kDa to 300 kDa.
The preferred molar ratio of toxin : carrier is 1-15 : 1.
The preferred administration of the toxin conjugates is intraveneous or intraperitoneal.
The preferred application of the toxin conjugates is the therapy of tumors and inflammation.
Preferred dose for humans is 10-100 μg/kg body weight.
Preferred application is 1 mg toxin /100 ml 0.9 % NaCl.

The toxin conjugates are also applied as tools in cell biology research. Both the amatoxins and the phallotoxins exert their toxic activity as described above only in the cytoplasm or in the nucleus of cells and for this have to pass through the cell membrane. Toxic effects in the cell are therefore used to indicate the internalization of the macromolecule employed into the cell.

Albumin-conjugates of amatoxins are also disclosed. They preferentially affect protein-consuming cells like sinusoidal cells of liver or macrophages (Barbanti-Brodano and Fiume, Nature New Biol. 1973, 243, 281-3). In mixtures of macrophages and lymphocytes, for example, albumin-ß-amanitin selectively extinguished the macrophages and left the lymphocytes intact. On the other hand, albumin-ß-amanitin was tolerated in rats in concentrations allowing the formation of amanitin-specific antibodies in rats. In similar experiments, amanitin-specific antibodies were produced in rabbits, by using fetuin-β-amanitin conjugates. The ratios of toxin : carrier used in the prior art were 3 - 17 : 1.

It has now been found that the toxicity of albumin-amantin conjugates can be largely diminished when the coupling conditions were changed. We prepared a human serum albumin β-amanitin conjugate by using β-amanitin-N-hydroxysuccinimide ester, and not carbodiimides and not the mixed anhydride method, together with a much lower ratio of toxin : albumin. When the concentration of amanitin (c) in such albumin conjugates was determined by u.v. spectrometry using the formula:

$$c_{amanitin} \ [\mu g/ml] = 1/13.5 \times ( E_{310} - 0.05 \ E_{280} ) \times 918$$

(where $E_{310}$ and $E_{280}$ are the absorbance values of the albumin conjugate at 300nm and 280 nm, respectively), it was found that on the average ca. 1 toxin molecule was linked to albumin. This procedure, together with the careful avoidance of denaturation and/or dimerization of the albumin component, reduced the toxicity of the new amanitin- albumin conjugates in mice by a factor of 10, related to previous preparations of ß-amanitin albumin conjugates.

For the preparation of an amatoxin-albumin conjugate 2 $\mu$mol of ß-amanitin (dried *in vacuo* over $P_4O_{10}$) was dissolved in 20$\mu$L of dimethylformamide. To this solution 14 $\mu$mol of solid N-hydroxy-succinimide were added and 3.6 $\mu$mol of solid dicyclohexylcarbodiimide. The mixture was allowed to react for 12 h at room temperature, and the crystallized dicyclohexylurea separated. The β-amanitin-N-hydroxysucinimide ester was precipitated by the addition of 1-2 mL of dry diethylether, and the precipitate isolated by centrifugation. Dissolved in 2.2 mL of human serum albumin (10% in PBS, 3.2 $\mu$mol), the mixture was gently shaken at 4°C over night. Unreacted ß-amanitin-N-hydroxysucinimide ester was separated from the toxin-albumin conjugate by gel filtration on Sepadex G25, using PBS as solvent. The solution was concentrated by ultrafiltration in centrifuge cones, sterilized, and kept at 4°C. The ratio toxin: albumin was in the range of 0.5 - 1 : 1.

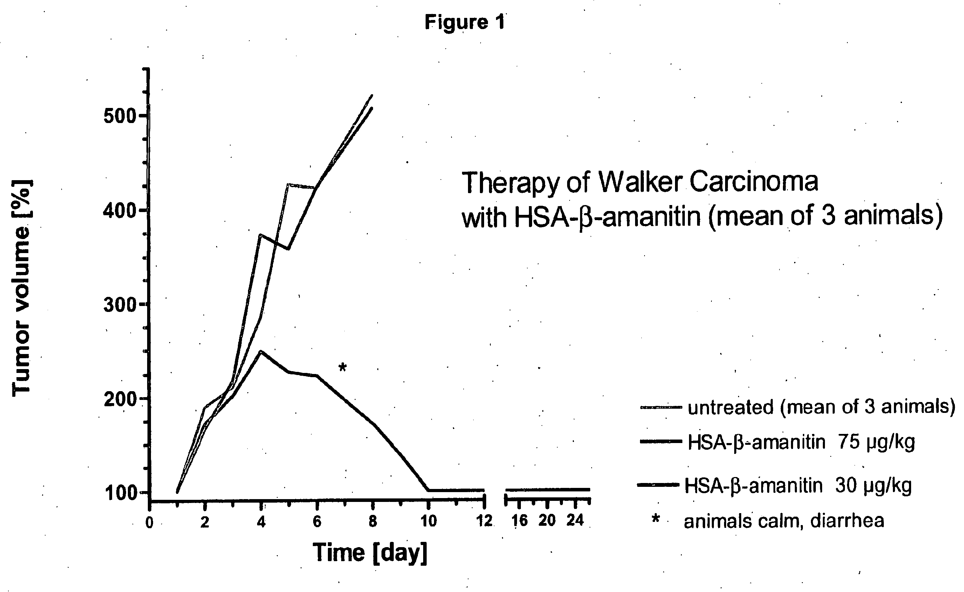

The novel albumin-ß-amanitin conjugate was tested for its anti-tumor activity using the Walker-256 Carcinoma in rat as a tumor model. Cells were cultivated in RPMI 1640 medium containing 10% fetal calf serum and 2mM glutamine, and splitted 2 times a week (1:2). The cells were centrifuged in PBS, washed and transplanted (5x10$^6$ cells/200$\mu$l) into one hind leg of female Sprague-Dawley rats. The tumor was palpable after 4-5 days when therapy was started. Animals received 75$\mu$g/kg bodyweight (75$\mu$g of β-amanitin bound to albumin) intravenously 2 times a week. After 3 injections the therapy was stopped because the animals lost weight and suffered from diarrhea. Tumor growth was measured every day and is shown in Fig. 1. While control animals showed an increase of tumor volume to 500% on day 9 (at that time the control animals were sacrificed because the leg became paralysed), 2/3 of the amanitin-treated animals stopped tumor growth on day 4 at a tumor volume of 220-240%. On day 6 the tumors of these animals softened and disappeared on day 10. The surviving animals were observed for further 14 days during which they recovered completely. They regained normal body weight and lived until sacrificed (further 2 weeks) without reappearance of the tumor. Another group of animals treated with a dose of 30$\mu$g/kg bodyweight showed no effect. These animals were sacrificed on day 9 together with the control animals (Fig.1).

The tumor-specific activity of albumin-bound amanitin becomes understandable by considering two observations made recently with solid tumors: 1. By the fact that solid tumors, particularly fast growing tumors, suffer from a deficiency of nutrients, amino acids, etc. and therefore absorb proteins, particularly albumin, from the surrounding tissue much more efficiently than normal cells. 2: By the fact that solid tumors cause inflammation reactions in their neighbourhood leading to fenestration of capillaries and an outflow of proteins from plasma into tissue (enhanced permeability and retention, EPR). Both effects contribute to a preferential uptake of albumin by solid tumors. While doted with amanitin, albumin becomes a vehicle for the toxin to be delivered into tumor cells. Once inside the cell, the vehicle is degraded by proteases in the endosomal/lysosomal compartment and the toxin, which is completely resistant to these enzymes, is released into cytoplasm in the active form. Evidence for such an albumin-supported delivery of ligands into solid tumors was given by H. Kremer, who showed by whole body scintigraphy of rats that an albumin DTPA- "In conjugate was accumulated in the Walker -256 Carcinoma (20% of the dose applied), while the rest of the body was blank, except for the region of heart/liver, representing 80% of the radioactivity 2 days after i.v. administration (H. Kremer, Habilitationsschrift, Heidelberg, 2002). By the same preference, an albumin conjugate with fluorescein is being successfully employed to differentiate, under u.v. light, tumor tissue from normal tissue in the brain before and during neurosurgery. (H.Kremer, Habilitationsschrift, Heidelberg, 2002).

Tumor-specific delivery of drugs by coupling of the drugs to albumin was extensively studied in the laboratory of G. Stehle, H. Sinn, and A. Wunder. These workers showed for methotrexate and several other well established tumor drugs that uptake of these drugs by solid tumors was increased, and, in turn, general toxicity decreased, when the drugs were conjugated to albumin (Eschen, N.,et al., Int. J. Clin. Pharmacol. Ther., 2002, 40:564-6;

Weigand, M., et al., Anticancer Drug Des., 2001, 16, 227-37; Schlicker, A., et al., PDA J. Pharm. Sci. Technol., 2000, 54, 442-8; Stehle, G., et al., Anticancer Drugs, 1999, 10, 785-90; Wunder et al., Int. J. Cancer 1998, 10, 884-90). The work presented here differs from the previous findings in that methotrexate, cis-platinum, and all other drugs used by Sinn and coworkers are tumor drugs by themselves. Coupling of these drugs to albumin modulates the pharmacokinetics of these drugs and by this reduces general toxicity. In contrast to this, amanitin is not a tumor drug. It attains this novel quality for tumor therapy only in conjugates with albumin or other macromolecules.

In addition to proteins like albumin or serum globulins, which do not find specific receptors on the surface of tumor cells, it is herein disclosed how to use therapeutically the conjugates of amatoxins and phallotoxins with proteins, which tightly bind to receptors on the cell membrane of tumor cells, as e.g. monoclonal antibodies raised against specific surface structures on tumor cells.

Monoclonal antibodies were conjugated with ß-amanitin-N-hydoxysuccinimide ester by incubating the antibody in PBS (2mg/mL) with 10-30 equivalents of the activated ß-amanitin derivative at 4°C under gentle shaking. After reaction for 16h the protein derivative was separated from unreacted amanitin by gelfiltration chromatography on Sephadex G25 using PBS as solvent. The combined protein fractions were concentrated to a protein concentration of ca 2 mg/mL and a toxin concentration of ca. $5 \times 10^{-5}$M. The ratio toxin : mAB was in the range of 5-15 : 1.

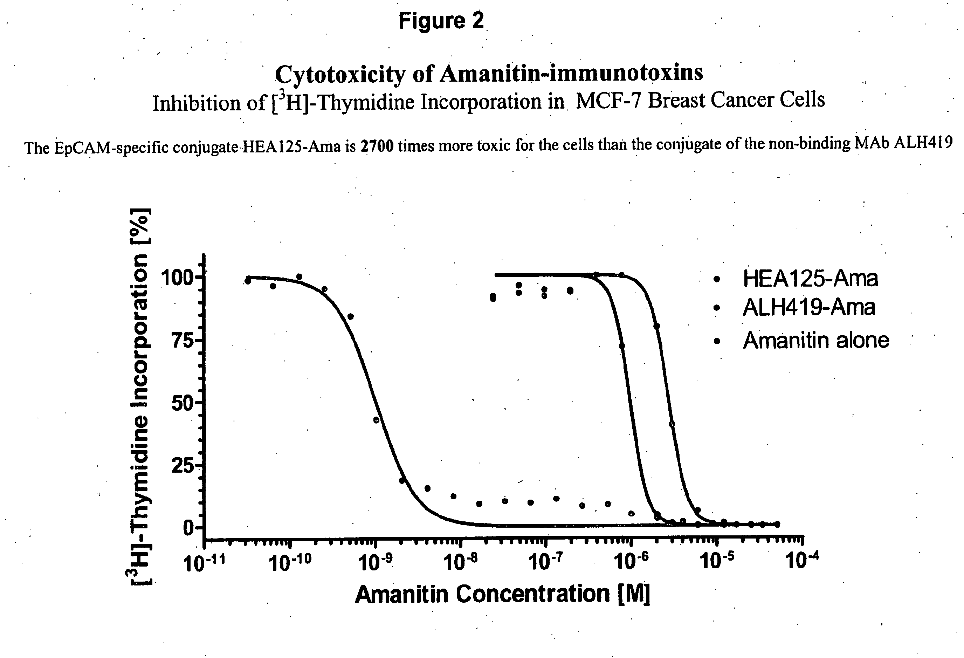

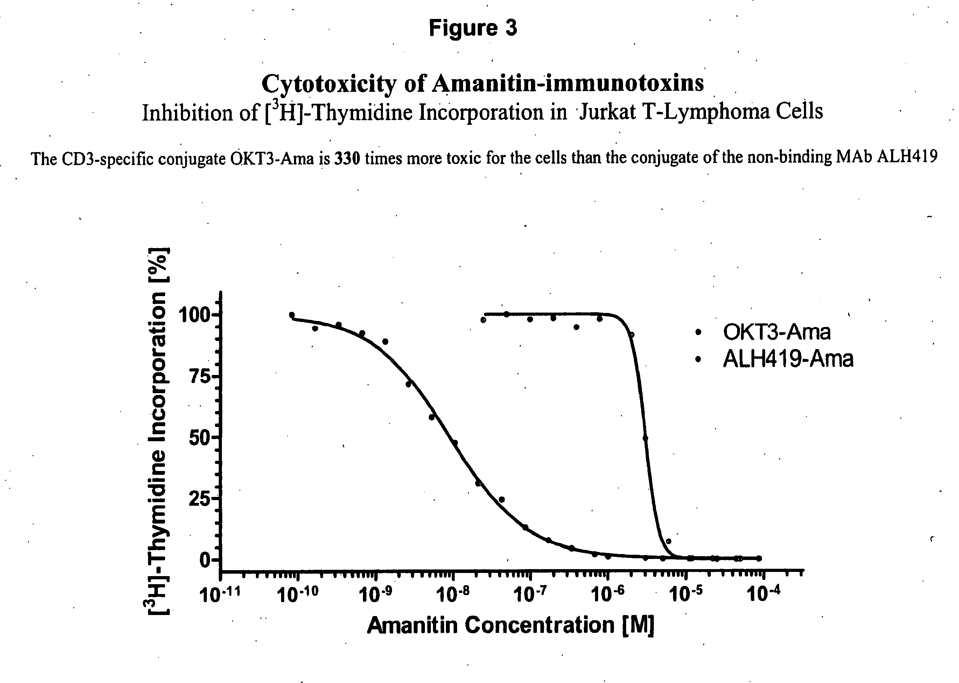

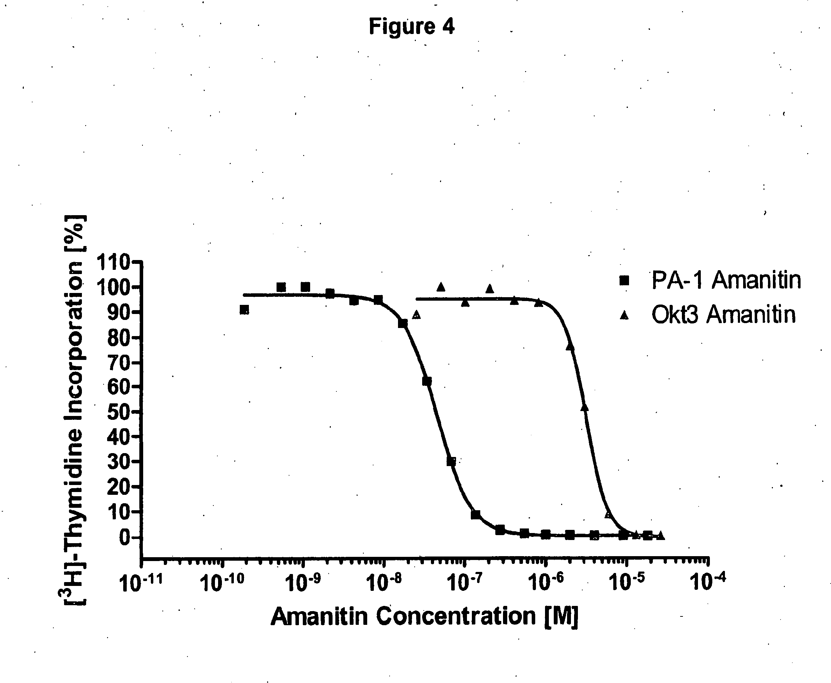

Three different cell lines, MCF-7, Jurkat, and Raji, exposing the specific receptors EpCAM, CD3, and CD71, respectively, were tested for the inhibition of proliferation by such ß-amanitin-monoclonal antibody conjugates, as followed by [$^3$H]thymidine incorporation. It was proved that the amanitin conjugate of the monoclonal antibody specific for the EpCAM receptor (HEA 125) was 2700times more inhibitory for the proliferation of MCF-7 cells than the amanitin conjugate of an unspecific, monoclonal antibody (ALH419), (Fig.2). Likewise, was the amanitin conjugate of the monoclonal antibody specific for the CD3 receptor (OKT3), 330 times more inhibitory for the proliferation of Jurkat cells than the amanitin conjugate of an unspecific, monoclonal antibody (ALH-419), (Fig.3) Finally we found that the amanitin conjugate of the monoclonal antibody against the CD71 receptor (PA-1) was 70 times more inhibitory for the proliferation of Raji cells than the amanitin conjugate of a monoclonal antibody specific for CD3, which is absent on Raji cells (Fig.4). In all cases was amanitin conjugated to a specific antibody distinctly more toxic to the corresponding cell line than free amanitin, or amanitin conjugated to unspecific antibodies.

For the preparation of the macromolecular derivatives of the phallotoxins, namely phalloidin and phallacidin, 2.0 mmol of toluene-4-sulfonic chloride was dissolved in 400 $\mu$l chloroform and added to 0.127 mmol of phalloidin or phallacidin dissolved in 1 ml ice-cold pyridine, drop by drop under stirring. After 30 min the reaction was stopped by the addition of 20 ml diethylether. The precipitate was washed with another 20 ml of diethylether and separated by chromatography on Sephadex LH20 with an overall yield of 68 %. For reaction with ammonia, 0.42 mmol monotosyl-phalloidin or monotosyl-phallacadin were reacted with 40 ml ice cold ammonia in methanol (2.5 N), and evaporated after 120 min. Yield of aminophalloidin and amino-phallacidin was ca. 90%. For coupling to macromolecules the toxin derivatives bearing the functional amino group were reacted with the homobifunctional crosslinking reagent dithiobis-(succinimidylpropionate, DSP). For this, 248 $\mu$moles DSP were dissolved in 1.0 ml dimethylformamide and added to 63 $\mu$moles of dried aminophalloidin. Reaction was started with 2 $\mu$l of triethylamine and proceeded under magnetic stirring for 16h at RT. The reaction product was precipitated with 10 ml diethylether and centrifugated, the sediment dissolved in 5 ml methanol and developed on a Sephadex-LH20 column using methanol. Yield of DSP-phalloidin or DSP-phallacidin was about 80 %, purity was minimum 90 %. For reaction with the macromolecules, 7 mg DSP-phalloidin was dissolved in 0.5 ml dimethylformamide and added to the macromolecule dissolved in 0.5 ml PBS. After reaction for 16h at RT, high-molecular weight products were separated by gelfiltration chromatography on Sephadex G25 using 0.1% NaCl as solvent. After lyophilisation, yield was determined by measuring the characteristic absorption of phalloidin and phallacidin at 300 nm ($\varepsilon$=10100). We calculated that with polylysine, for example, one out of 10 lysine residues was conjugated with aminophalloidin or aminophallacidin.

The cytotoxicity of phallotoxin conjugates was assessed by using the 3-(4,5-dimethylthiazol-2-yl)-2,5-diphenyltetrazolium bromide (MTT) assay. Mouse fibroblasts MF/LDB, NIH/3T3, or Hela maintained in Dulbecco's modified minimal essential medium containing 10% fetal calf serum, as well as Jurkat cells mantained in RPMI 1640 medium containing 10% fetal calf serum and 0,05 mM β-mercaptoethanol, were cultured at 37°C in a humidified 95% air / 5% $CO_2$ incubator. Exponentially growing cells were plated at a density of $2 \times 10^4$ cells/well in 96 well plates 24h before the toxin was added using a volume equal to the volume of medium in the culture dish. The final concentration of toxins in media was between $10^{-3}$ M and $10^{-9}$ M. At 72h, the medium was replaced by serum-free medium containing 25 $\mu$l MTT solution (5 mg/ml in PBS) and the incubation continued at 37°C for 4 h. Then lysis buffer (100 $\mu$l 20% SDS in 50% dimethylformamide) was added to each well and incubated for another 16-20h. Viability of cells was determined by measuring the 570nm absorbance of each well using a microplate reader (Molecular Devices). $IC_{50}$ values were calculated as the concentration of toxin required to reduce the absorbance to 50% of control cultures.

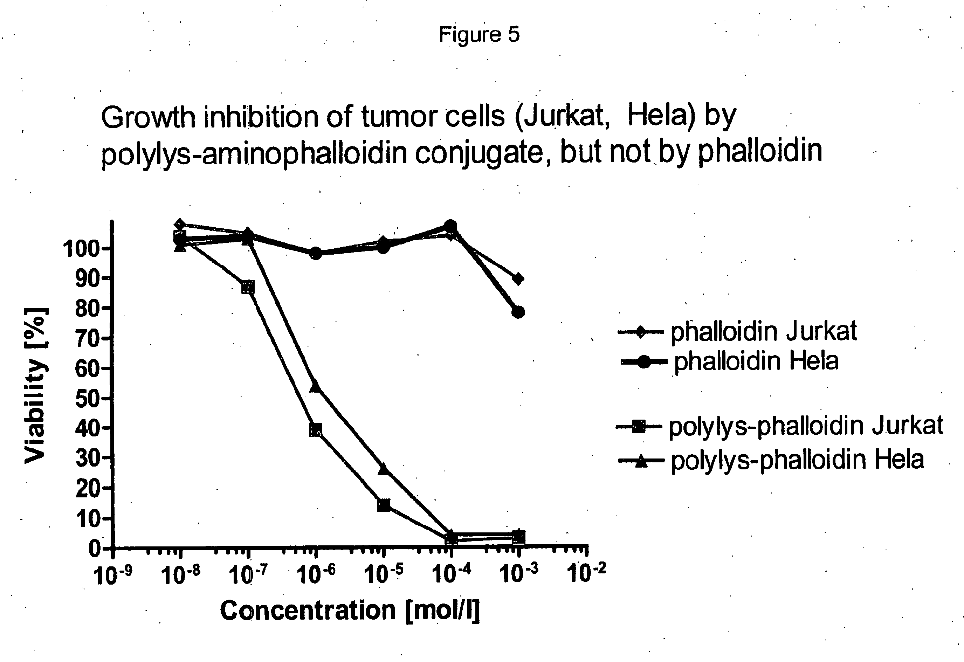

In both Jurkat and Hela cells the macromolecular conjugates of aminophalloidin were distinctly more toxic to tumor cells (>1000) than the native phalloidin. The data are shown in Fig.5. Of high importance for this cytotoxic activity is the presence of a disulfide group in the linker moiety between toxin and macromolecule. After uptake into the tumor cells the disulfide group is cleaved by reduction releasing thiopropionyl-aminophalloidin or thiopropionyl-aminophallacidin into cytoplasm. We showed that thiopropionyl-aminophalloidin binds actin as tightly as phalloidin itself. This suggests that also its cytotoxic activity inside the cell is in the range of that of phalloidin.

Possible benefit of the present invention is to make available for therapeutic use agents like amatoxins which so far were excluded from therapeutical use, but impress by their ability to induce apoptosis in tumor cells or in noxious cells of the lymphatic system, when conjugated to suitable macromolecular carriers. Both, the treatment of solid tumors and of inflammatory diseases by such macromolecular conjugates takes advantage of the fact that inflammatory processes as well as solid tumors cause a leakage of the capillary system (fenestration, enhanced permeability and retention, EPR-effect). This effect allows macromolecules (e.g. proteins) to penetrate into the surrounding tissue, thus increasing the concentration of serum macromolecules in the environment of tumors and at sites of inflammation. This may explain the specific cytotoxicity observed in cases where no specific receptors for a conjugate are available. Moreover, solid tumors suffer from a short supply of amino acids and glucose and therefore internalize serum proteins (e.g. albumin) in much higher amounts than normal cells to meet their requirements of metabolites and energy. Both effects are important for the successful application of the described toxin conjugates in the treatment of tumor and inflammation diseases.

**Claims**

1. Conjugate of the general formula carrier-toxin, wherein

    - the toxin is an amatoxin; and
    - the carrier comprises a tumorspecific monoclonal antibody which binds to or is taken up by tumour cells;
    wherein the conjugate is obtainable by reacting the tumorspecific monoclonal antibody with 10 to 30 equivalents β-amanitin-N-hydroxysuccinimideester for 16 h at 4°C.

2. The conjugate of claim 1, wherein the carrier has a molar mass from 20 kDa to 300 kDa.

3. The conjugate of any one of claims 1 to 2, wherein the molar ratio toxin : carrier is between 1:1 and 15:1.

4. Composition comprising the conjugate of any one of claims 1 to 3 in combination with further anti-cancer compounds.

5. The composition of claim 4, wherein the further anti-cancer compounds are selected from the group consisting of nucleoside analogs, taxol, and tecans.

6. Pharmaceutical composition comprising

    (a) the conjugate of any one of claims 1 to 3; or
    (b) the composition of any one of claims 4 to 5
    in combination with a pharmaceutically acceptable carrier.

7. The conjugate of any one of claims 1 to 3 or the composition of any one of claims 4 to 6 for therapeutic use.

8. The conjugate of any one of claims 1 to 3 or the composition of any one of claims 4 to 6 for use in the treatment of cancer.

9. Use of the conjugate of any one of claims 1 to 3 or the composition of any one of claims 4 to 6 for the preparation of a medicament for the treatment of cancer.

10. Use of the conjugate of any one of claims 1 to 3 in cell biology assays to indicate the internalization of the conjugate, comprising the steps of:

    - *in vitro* incubation of said conjugate in the presence of target cells; and
    - determining the appearance of toxic events in the cell.

11. The use of claim 10, wherein the toxic event is apoptosis.

**Patentansprüche**

1. Ein Konjugat der allgemeinen Formel Träger - Toxin, wobei

   - das Toxin ein Amatoxin ist; und
   - der Träger einen tumorspezifischen monoklonalen Antikörper umfasst, der an Tumorzellen bindet oder von Tumorzellen aufgenommen wird;
   wobei das Konjugat durch Reagieren des tumorspezifischen monoklonalen Antikörpers mit 10 bis 30 Äquivalenten β-amanitin-N-hydroxysuccinimidester für 16 Stunden bei 4°C erhältlich ist.

2. Das Konjugat des Anspruchs 1, wobei der Träger eine molare Masse von 20 kDa bis 300 kDa hat.

3. Das Konjugat eines der Ansprüche 1 bis 2, wobei das molare Verhältnis Toxin - Träger zwischen 1:1 und 15:1 ist.

4. Eine Zusammensetzung umfassend das Konjugat eines der Ansprüche 1 bis 3 in Kombination mit weiteren Anti-Krebs Verbindungen.

5. Die Zusammensetzung des Anspruchs 4, wobei die weiteren Anti-Krebs Verbindungen aus der Gruppe bestehend aus Nucleosid-Analoga, Taxol und Tecane ausgewählt sind.

6. Eine pharmazeutische Zusammensetzung umfassend

   (a) das Konjugat eines der Ansprüche 1 bis 3; oder
   (b) die Komposition eines der Ansprüche 4 bis 5
   in Kombination mit einem pharmazeutisch verträglichem Träger.

7. Das Konjugat eines der Ansprüche 1 bis 3 oder die Komposition eines der Ansprüche 4 bis 6 für eine therapeutische Verwendung.

8. Das Konjugat eines der Ansprüche 1 bis 3 oder die Komposition eines der Ansprüche 4 bis 6 für die Verwendung in der Behandlung von Krebs.

9. Verwendung des Konjugats eines der Ansprüche 1 bis 3 oder der Komposition eines der Ansprüche 4 bis 6 für die Zubereitung eines Medikaments für die Behandlung von Krebs.

10. Verwendung des Konjugats eines der Ansprüche 1 bis 3 in zellbiologischen Assays, um die Internalisierung des Konjugats zu zeigen, umfassend die Schritte:

    - *in vitro* Inkubation des Konjugats in Anwesenheit von Zielzellen; und
    - Bestimmen von Anzeichen toxischer Vorgänge in der Zelle.

11. Die Verwendung gemäß Anspruch 10, wobei der toxische Vorgang Apoptose ist.


**Revendications**

1. Conjugué de formule générale support-toxine, dans laquelle

   - la toxine est une amatoxine; et
   - le support comprend un anticorps monoclonal spécifique pour une tumeur, qui se lie à ou est internalisé par des cellules tumorales;
   tandis que le conjugué peut être obtenu par réaction de l'anticorps monoclonal spécifique pour une tumeur avec 10 à 30 équivalents d'ester β-amanitine-N-hydroxysuccinimidique pendant 16 h à 4°C.

2. Conjugué selon la revendication 1, dans lequel le support a une masse molaire de 20 kDa à 300 kDa.

3. Conjugué selon l'une quelconque des revendications 1 à 2, dans lequel le rapport molaire toxine:support est entre 1:1 et 15:1.

**4.** Composition comprenant le conjugué selon l'une quelconque des revendications 1 à 3 en combinaison avec d'autres composés anticancéreux.

**5.** Composition selon la revendication 4, dans laquelle les autres composés anticancéreux sont choisis dans le groupe consistant en analogues nucléosidiques, taxol, et técans.

**6.** Composition pharmaceutique comprenant:

(a) le conjugué selon l'une quelconque des revendications 1 à 3; ou
(b) la composition selon l'une quelconque des revendications 4 à 5 en combinaison avec un support pharmaceutiquement acceptable.

**7.** Conjugué selon l'une quelconque des revendications 1 à 3 ou composition selon l'une quelconque des revendications 4 à 6 pour utilisation thérapeutique.

**8.** Conjugué selon l'une quelconque des revendications 1 à 3 ou composition selon l'une quelconque des revendications 4 à 6 pour utilisation dans le traitement du cancer.

**9.** Utilisation du conjugué selon l'une quelconque des revendications 1 à 3 ou de la composition selon l'une quelconque des revendications 4 à 6 pour la préparation d'un médicament pour le traitement du cancer.

**10.** Utilisation du conjugué selon l'une quelconque des revendications 1 à 3 dans des essais en biologie cellulaire pour indiquer l'internalisation du conjugué, comprenant les étapes de:

- l'incubation *in vitro* dudit conjugué en présence de cellules cibles; et
- la détermination de l'apparence des évènements toxiques dans la cellule.

**11.** Utilisation selon la revendication 10, dans laquelle l'évènement toxique est l'apoptose.

Figure 1

Therapy of Walker Carcinoma
with HSA-β-amanitin (mean of 3 animals)

———— untreated (mean of 3 animals)

———— HSA-β-amanitin  75 µg/kg

———— HSA-β-amanitin  30 µg/kg

*    animals calm, diarrhea

## Figure 2

### Cytotoxicity of Amanitin-immunotoxins
Inhibition of [$^3$H]-Thymidine Incorporation in MCF-7 Breast Cancer Cells

The EpCAM-specific conjugate HEA125-Ama is 2700 times more toxic for the cells than the conjugate of the non-binding MAb ALH419

# Figure 3

## Cytotoxicity of Amanitin-immunotoxins
Inhibition of [$^3$H]-Thymidine Incorporation in Jurkat T-Lymphoma Cells

The CD3-specific conjugate OKT3-Ama is **330** times more toxic for the cells than the conjugate of the non-binding MAb ALH419

Figure 4

Figure 5

## Growth inhibition of tumor cells (Jurkat, Hela) by polylys-aminophalloidin conjugate, but not by phalloidin

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WIELAND, T. ; FAULSTICH H.** *Crit. Rev. Biochem.,* 1978, vol. 5, 185-260
- **BARBANTI-BRODANO ; FIUME.** *Nature New Biol.,* 1973, vol. 243, 281-3
- **H. KREMER.** Habilitationsschrift. 2002
- **H.KREMER.** Habilitationsschrift. 2002
- **ESCHEN, N.** *Int. J. Clin. Pharmacol. Ther,* 2002, vol. 40, 564-6
- **WEIGAND, M et al.** *Anticancer Drug Des,* 2001, vol. 16, 227-37
- **SCHLICKER, A. et al.** *PDA J. Pharm. Sci. Technol,* 2000, vol. 54, 442-8
- **STEHLE, G et al.** *Anticancer Drugs,* 1999, vol. 10, 785-90
- **WUNDER et al.** *Int. J. Cancer,* 1998, vol. 10, 884-90